# EUROPÄISCHE PATENTANMELDUNG

(11) **EP 2 446 910 A1**
(43) Veröffentlichungstag der Anmeldung: **02.05.2012**
(21) Anmeldenummer: 10188987.1
(22) Anmeldetag: 27.10.2010
(51) Int. Cl.: A61M 5/142

(54) **Pumpe und Verfahren zum Fördern eines Fluids sowie Hohlkörper für eine Pumpe**

(71) Anmelder: Fresenius Kabi Deutschland GmbH, 61352 Bad Homburg (DE)
(72) Erfinder: Neumann, Dr. Hans-Jürgen, 66606, St. Wendel (DE); Meisberger, Artur, 66606, St. Wendel (DE); Akdis, Mustafa, 35799, Merenberg (DE)
(74) Vertreter: Richly, Erik

(57) **Zusammenfassung**

Die Erfindung betrifft eine Pumpe zum Fördern eines Fluids in einem Hohlkörper, mit
- einer Aktuatorvorrichtung (4), die bei Betrieb der Pumpe (1) einen ersten, zweiten und dritten Abschnitt (31-33, 33', 71-73) einer flexiblen Wandung des Hohlkörpers (3, 7) gemäß einer vorgegebenen und periodisch wiederholten Fördersequenz aus einer Ausgangsstellung in eine Endstellung bewegt, wobei
- ein Bereich des Hohlkörpers (3, 7) zwischen dem ersten und dem dritten Abschnitt (31, 33', 71, 33, 73) der flexiblen Wandung eine Förderkammer (35, 75) darstellt, aus der durch Bewegen des zweiten Abschnitts (32, 72) in die Endstellung Fluid (2) herausgedrückt wird.

Erfindungsgemäß ist die Aktuatorvorrichtung (1) ausgebildet, einen vierten Abschnitt (34, 74) der flexiblen Wandung, der sich entlang der Förderrichtung (A) vor dem dritten Abschnitt (33, 33', 73) befindet, aus einer Ausgangsstellung in eine Endstellung zu bewegen, und zum Erzeugen eines möglichst reproduzierbaren Druckes in der Förderkammer (35) den dritten Abschnitt (33, 33', 73) in Endstellung zu bewegen, während sich sowohl der vierte als auch der erste Abschnitt (34, 74, 31, 71) in Endstellung befinden, so dass durch das Bewegen des dritten Abschnitts (33, 33', 73) in die Endstellung Fluid (2) in die Förderkammer (35, 75) gedrückt wird.

Die Erfindung betrifft auch ein Verfahren zum Fördern eines Fluids. Des Weiteren betrifft die Erfindung einen Hohlkörper für eine Pumpe.

## Beschreibung

Die Erfindung betrifft eine Pumpe zum Fördern eines Fluids gemäß dem Oberbegriff des Anspruchs 1, einen Hohlkörper für eine Pumpe gemäß Anspruch 14 sowie ein Verfahren zum Fördern eines Fluids gemäß dem Oberbegriff des Anspruchs 15.

Pumpen, die über ein periodisches Eindrücken einer flexiblen Wandung eines Hohlkörpers ein Fluid fördern, sind z. B. in Form von Schlauchpumpen aus dem Stand der Technik bekannt. Des Weiteren sind Membranpumpen bekannt, die durch Eindrücken einer Membran ein Fluid in einem durch die Membran verschlossenen Hohlkörper fördern. Die Langzeitstabilität der Förderrate derartiger Pumpen ist vielfach dadurch eingeschränkt, dass sich die Eigenschaften des Schlauches bzw. der Membran aufgrund der Beanspruchung während des Pumpens mit der Zeit verändern.

Das der Erfindung zugrunde liegende Problem besteht darin, eine Pumpe zum Fördern eines Fluids sowie einen Hohlkörper für eine Pumpe zu schaffen, die eine möglichst gleichbleibende Förderrate sowie einen vom Eingangsdruck möglichst unabhängigen Ausgangsdruck über einen möglichst langen Zeitraum ermöglichen. Des Weiteren soll ein entsprechendes Verfahren zur Verfügung gestellt werden.

Diese Probleme werden durch die Vorrichtung mit den Merkmalen des Anspruchs 1, durch den Hohlkörper gemäß Anspruch 14 sowie durch das Verfahren mit den Merkmalen gemäß Anspruch 15 gelöst. Weiterbildungen der Erfindung sind in den abhängigen Ansprüchen angegeben.

Danach wird eine Pumpe zum Fördern eines Fluids in einem Hohlkörper bereitgestellt, mit
- einer Aktuatorvorrichtung, die bei Betrieb der Pumpe einen ersten, zweiten und dritten Abschnitt einer flexiblen Wandung des Hohlkörpers gemäß einer vorgegebenen und periodisch wiederholten Fördersequenz aus einer Ausgangsstellung in eine Endstellung bewegt, wobei
- sich der Strömungsquerschnitt des Hohlkörpers im Bereich des ersten, zweiten oder dritten Abschnitts verringert, wenn der entsprechende Abschnitt in seine Endstellung bewegt wird, so dass der Strömungsquerschnitt des Hohlkörpers im Bereich dieser Abschnitte gemäß der Fördersequenz periodisch verringert und auf diese Weise in dem Hohlkörper Fluid entlang einer Förderrichtung gefördert wird,
- ein Bereich des Hohlkörpers zwischen dem ersten und dem dritten Abschnitt der flexiblen Wandung eine Förderkammer darstellt, aus der durch Bewegen des zweiten Abschnitts in Endstellung Fluid herausgedrückt wird, und wobei
- die Aktuatorvorrichtung ausgebildet ist,
einen vierten Abschnitt der flexiblen Wandung, der sich entlang der Förderrichtung vor dem dritten Abschnitt befindet, aus einer Ausgangsstellung in eine Endstellung zu bewegen, und
zum Erzeugen eines möglichst reproduzierbaren Druckes in der Förderkammer den dritten Abschnitt in Endstellung zu bewegen, während sich sowohl der vierte als auch der erste Abschnitt in Endstellung befinden, so dass durch das Bewegen des dritten Abschnitts in die Endstellung Fluid in die Förderkammer gedrückt wird.

Die "Fördersequenz" legt insbesondere die Reihenfolge fest, in der der erste, zweite, dritte und vierte Abschnitt aus der Ausgangstellung in die Endstellung bewegt werden und z.B. auch, wie lange die Abschnitte jeweils in dieser Position verbleiben. Selbstverständlich müssen der erste, zweite, dritte und vierte Abschnitt nicht (in dieser Reihenfolge) nacheinander in die Endstellung bewegt werden. Vielmehr wird z.B. der vierte Abschnitt - innerhalb einer Fördersequenz (d.h. eines Pumpzykus) - vor dem ersten Abschnitt bewegt.

Des Weiteren wird darauf hingewiesen, dass die "Endstellung" eines Abschnittes der flexiblen Wandung zwar durchaus eine Position des Abschnittes bezeichnen kann, in der der Strömungsquerschnitt des Hohlraums im Wesentlichen komplett verschlossen ist, d.h. eine Sperrstellung. Die Endstellung eines Abschnitts kann jedoch auch schon vorher erreicht sein, so dass z.B. ein Restquerschnitt geöffnet bleibt, durch den eine gewisse Menge Fluid hindurchströmen kann.

Der erste und der dritte Abschnitt der flexiblen Hohlkörperwandung sind so beabstandet zueinander positioniert, dass der Hohlkörper zwischen ihnen eine Kammer (Förderkammer) ausbildet, aus der Fluid durch Bewegen des zweiten Abschnittes in seine Endstellung herausgedrängt und somit in Förderrichtung (auf den ersten Abschnitt zu) bewegt werden kann. Es wird darauf hingewiesen, dass die Formulierung, wonach die Förderkammer "zwischen" dem ersten und dem dritten Abschnitt ausgebildet ist, nicht unbedingt bedeutet, dass sich die Förderkammer von dem ersten bis zu dem dritten Abschnitt erstreckt. Es ist jedoch denkbar, dass die Aktuatorvorrichtung als zweiten Abschnitt einen Bereich der flexiblen Wandung bewegt, der sich über mehr als die Hälfte des Abstandes zwischen dem ersten und dem dritten Abschnitt erstreckt. Insbesondere ist natürlich auch möglich, dass sich der zweite Abschnitt über nahezu den gesamten Abschnitt zwischen dem über die Aktuatorvorrichtung bewegten ersten und dritten Abschnitt der flexiblen Wandung erstreckt. Auf das Pumpprinzip der Pumpe wird noch weiter unten im Detail eingegangen.

Über das Bewegen des vierten Abschnitts, der - in Strömungsrichtung betrachtet - vor den anderen mittels der Aktuatorvorrichtung bewegbaren Abschnitten liegt, in seine Endstellung wird ein Einströmen von Fluid unterbunden bzw. zumindest reduziert, d.h. der vierte Abschnitt ist gewissermaßen Bestandteil eines "Einlassventils" der Pumpe. Entsprechend kann der erste Abschnitt als Bestandteil eines "Auslassventils" angesehen werden, da er in Endstellung einem Ausströmen von Fluid aus dem Bereich der Pumpe (d.h. aus dem Bereich des Hohlkörpers zwischen dem vierten und dem ersten Abschnitt) entgegenwirkt bzw. komplett unterbindet.

Durch dieses Verschließen des Zulaufs und des Ablaufs der Pumpe kann das zwischen dem vierten und dem ersten Abschnitt (d.h. zwischen dem Einlass- und dem Auslassventil) im Hohlkörper vorhandene Fluid nicht aus der Pumpe hinausströmen, wenn der dritte Abschnitt des Hohlkörpers in seine Endstellung gebracht und das in seinem Bereich vorhandene Fluid verdrängt wird. Vielmehr strömt zumindest ein Teil des verdrängten Fluids in die Förderkammer, so dass dort vor dem Bewegen des zweiten Abschnitts (d.h. dem Herausdrücken des Fluids aus der Förderkammer) ein reproduzierbarer Vordruck erzeugt wird. Dadurch ist es insbesondere möglich, über viele Pumpzyklen (d.h. Wiederholungen der Fördersequenz) hinweg eine reproduzierbare, insbesondere möglichst konstante Förderrate und Ausgangsdruck zu erzeugen, wobei die Förderrate und der Ausgangsdruck insbesondere auch möglichst unabhängig von einem am Pumpeneingang vorhandenen Eingangsdruck sind.

Die Aktuatorvorrichtung ist zudem natürlich so beschaffen, dass sie nicht nur das Bewegen der Abschnitte aus der Ausgangs- in die Endstellung gemäß der Fördersequenz bewirkt (z.B. durch Eindrücken der flexiblen Wandung im Bereich des jeweiligen Abschnitts), sondern auch das Zurückkehren des jeweiligen Abschnittes aus seiner End- in die Ausgangsstellung (insbesondere aufgrund einer Elastizität der flexiblen Hohlkörperwandung) ermöglicht. Insbesondere bewegt die Aktuatorvorrichtung die Abschnitte der flexiblen Wandung zwischen der Ausgangs- und der Endstellung periodisch hin und her. Auf konkrete Möglichkeiten zur Realisierung der Aktuatorvorrichtung wird weiter unten eingegangen.

Bei dem Fluid handelt es sich insbesondere um eine Flüssigkeit oder ein Gas, das mit der Pumpe und über den Hohlkörper (z.B. aus einem Reservoir) gefördert wird.

Die erwähnten vier Abschnitte der flexiblen Wandung des Hohlkörpers liegen insbesondere entgegen der Förderrichtung hintereinander, z.B. entlang einer Geraden, d. h. eine Gerade durchläuft sowohl den ersten, den zweiten, den dritten als auch den vierten Abschnitt. Es ist jedoch auch denkbar, dass zumindest einige der Abschnitte zwar in einer bestimmten Richtung betrachtet hintereinander liegen, jedoch versetzt zueinander angeordnet sind. Darüber hinaus ist es natürlich auch möglich, dass die Aktuatorvorrichtung ausgebildet ist, neben den beschriebenen vier Abschnitten weitere Abschnitte der flexiblen Wandung zu bewegen, z.B. weitere vier Abschnitte mit einer Funktion analog zu den beschriebenen Abschnitten.

Gemäß einer Ausgestaltung der Erfindung weist die Aktuatorvorrichtung ein erstes, zweites, drittes und viertes Andrückelement sowie Antriebsmittel auf, die jeweils dem ersten, zweiten, dritten und vierten Abschnitt zugeordnet sind, und über die das erste Andrückelement gegen den ersten Abschnitt, das zweite Andrückelement gegen den zweiten Abschnitt, das dritte Andrückelement gegen den dritten Abschnitt und das vierte Andrückelement gegen den vierten Abschnitt der flexiblen Wandung drückbar ist.

Insbesondere sind die Andrückelemente jeweils kolbenartig ausgebildet und können über die Antriebsmittel in einer zur flexiblen Wandung senkrechten Richtung hin- und herbewegt werden. Beispielsweise weisen die Antriebsmittel eine Nockenwelle auf, wobei jedem der Andrückelemente ein an dieser Nockenwelle festgelegter Nocken zugeordnet ist, so dass durch Rotation der Nockenwelle eine Hin- und Herbewegung der Andrückelemente zwischen einer Ausgangsposition und einer Endposition erzeugt wird. Insbesondere befinden sich die jeweiligen Abschnitte der flexiblen Wandung in ihrer Ausgangstellung, wenn das zugeordnete Andrückelement in seiner Ausgangsposition ist und entsprechend in Endstellung, wenn sich das Andrückelement in Endposition befindet. Derartige Antriebsmittel sind jedoch im Prinzip bekannt, so dass sie hier nicht weiter erläutert werden. Beispielsweise drücken die Andrückelemente mit einer dem jeweils zugeordneten Nocken abgewandten Seite gegen die flexible Wandung des Hohlkörpers.

Beispielsweise ist zwischen dem zweiten und dem dritten Andrückelement ein weiteres Andrückelement angeordnet, das einem weiteren Abschnitt der Wandung des Hohlkörpers zwischen dem zweiten und dem dritten Abschnitt zugeordnet ist und das nach Bewegen des dritten Andrückelementes in die Endstellung ebenfalls in seine Endstellung gebracht wird, wobei die Förderkammer durch das weitere Andrückelement und das erste Andrückelement (das Auslassventil) begrenzt ist. Insbesondere ist hier das dem dritten Abschnitt zugeordnete dritte Andrückelement über ein elastisches Element (z.B. in Form einer Feder) gelagert.

Das weitere Andrückelement hat z.B. eine Ventilfunktion, d.h. es verschließt nach Bewegen des dritten Andrückelementes in die Endstellung den Hohlkörper möglichst vollständig, um während des Förderschrittes (d.h. während des Bewegens des zweiten Abschnitts in die Endstellung) ein Herausfließen von Fluid aus der Förderkammer zu vermeiden.

Zwischen dem vierten Andrückelement (d.h. dem Einlassventil der Pumpe) und dem weiteren Andrückelement bildet der Hohlkörper eine Vorkammer aus, wobei durch die elastische Lagerung des im Bereich der Vorkammer befindlichen dritten Andrückelementes ein gewisses Ausdehnen des Hohlkörpers im Bereich der Vorkammer möglich ist, um zu vermeiden, dass der Druck in der Vorkammer zu groß wird und der Hohlkörper birst, wenn das weitere Andrückelement in seine Endstellung bewegt wird. Es ist auch denkbar, dass durch das Bewegen des weiteren Andrückelementes ebenfalls Fluid in die Förderkammer gedrückt wird, falls diese nach Bewegen des dritten Andrückelementes in die Endstellung noch nicht vollständig gefüllt ist.

Wie unten erläutert werden wird, ist es auch denkbar, dass das dritte Andrückelement nicht elastisch gelagert ist, sondern in Form eines Ventilstößels in Endposition den Hohlkörper möglichst vollständig verschließt. Beim Bewegen des Ventilstößels in die Endposition wird Fluid in die Förderkammer gedrückt, um dies möglichst reproduzierbar (d.h. z.B. in jedem Pumpzyklus zumindest näherungsweise vollständig) zu befüllen. Hier bildet der Hohlkörper zwischen dem vierten Abschnitt (dem Einlassventil der Pumpe) und dem dritten Abschnitt eine Vorkammer aus, wobei z.B. die Eigenelastizität der flexiblen Wandung im Bereich der Vorkammer ein Ausdehnen des Hohlkörpers ermöglicht, so dass verdrängtes Fluid, das nicht mehr in die Förderkammer einströmen kann, in der Vorkammer aufgenommen wird.

Die (entlang der Förderrichtung betrachtete) Breite der Andrückelemente kann unterschiedlich sein. In einer Variante weist das dem zweiten Abschnitt der flexiblen Wandung zugeordnete (zwischen dem ersten und dem dritten Andrückelement platzierte) zweite Andrückelement eine größere Breite auf als das dem ersten und/oder dem dritten Abschnitt zugeordnete erste bzw. dritte Andrückelement auf. Beispielsweise ist die Breite des zweiten Andrückelements so gewählt, dass es sich über einen Großteil des Abstandes zwischen dem dritten und dem ersten Andrückelement erstreckt, falls die Förderkammer durch diese Andrückelemente gebildet ist. Wie oben beschrieben, ist es auch denkbar, dass ein weiteres Andrückelement (insbesondere in Form eines Ventilstößels) zwischen dem zweiten und dem dritten Andrückelement angeordnet ist, wobei die Förderkammer von diesem weiteren Andrückelement und dem ersten Andrückelement begrenzt ist.

Beispielsweise erstreckt sich das zweite Andrückelement entlang der Förderrichtung über mindestens 70%, mindestens 85% oder mindestens 95 % des Abstandes zwischen den die Förderkammer begrenzenden Andrückelementen (d.h. zwischen dem ersten und dem dritten Andrückelement bzw. zwischen dem weiteren Andrückelement und dem ersten Andrückelement). Dadurch wird einem Ausdehnen des Hohlkörpers in Bereich au-βerhalb des zweiten Andrückelementes entgegengewirkt, wenn Fluid in die Förderkammer gedrückt wird, so dass eine möglichst vollständige Füllung der Förderkammer im Bereich des zweiten Andrückelementes realisiert werden kann. Darüber hinaus kann die Aktuatorvorrichtung so ausgebildet sein, dass sie das zweite Andrückelement vor Bewegen des dritten Andrückelementes in die Endstellung in Anlage mit dem Hohlkörper hält, um eine Ausdehnung der Förderkammer beim Einströmen von Fluid möglichst gering zu halten.

Es wird darauf hingewiesen, dass die Erfindung nicht auf eine bestimmte Art von Aktuatorvorrichtungen festgelegt ist. Anstelle oder zusätzlich zu den beschriebenen Andrückelementen und den entsprechenden Antriebsmitteln können auch andere Arten von Aktuatorvorrichtungen verwendet werden, z. B. eine nicht mechanische (berührungslose) Aktuatorvorrichtung (die insbesondere hydraulische, pneumatische, elektromagnetische oder thermische Mittel zur Bewegung der Hohlkörperabschnitte aufweist). Beispielsweise ist denkbar, dass die Aktuatorvorrichtung ein erstes magnetisches Element aufweist, das mit einem zweiten magnetischen Element wechselwirkt, das im Bereich des zu bewegenden Abschnitts an der flexiblen Wandung des Hohlkörpers angebracht ist.

Gemäß einer anderen Weiterbildung der Erfindung ist das vierte Andrückelement (das Einlassventil) über ein elastisches Element, insbesondere in Form einer Feder, gelagert, so dass es bei Überschreiten eines vorgebbaren Innendruckes in dem Hohlkörper (insbesondere in einer zwischen dem vierten Andrückelement und der Förderkammer gebildeten Vorkammer) aus seiner Endposition weg (in Richtung Ausgangsposition) bewegt wird, so dass sich der Strömungsquerschnitt des Hohlkörpers im Bereich des vierten Andrückelementes vergrößert. Das vierte Andrückelement realisiert somit gewisserma-βen ein druckreguliertes Ventil, so dass bei einem zu hohen Druck im Hohlkörper (insbesondere in der Vorkammer) Fluid abgeleitet werden kann. Dies vermeidet z.B. eine Beschädigung des Hohlkörpers (insbesondere der flexiblen Wandung) in dem Fall, dass das Volumen des Hohlkörpers außerhalb des dritten Abschnitts das bei Bewegen des dritten Andrückelementes (d.h. des dritten Abschnitts) in seine Endposition verdrängte Fluidvolumen nicht aufnehmen kann.

Die erfindungsgemäße Pumpe kann darüber hinaus Mittel zum Bestimmen des Innendrucks in der Förderkammer aufweisen, die insbesondere dazu dienen, Fehlerzustände, die die Fördergenauigkeit beeinträchtigen können, zu erkennen. Beispielsweise kann über eine Druckmessung in der Förderkammer ein Verschluss (Okklusion) einer Zuleitung zu der Pumpe, ein Verschluss einer von der Pumpe weg führenden Ausgangsleitung und/oder die ungewollte Förderung von Luft erkannt werden. Insbesondere ermöglicht eine Ermittlung des zeitlichen Druckverlaufs über eine Fördersequenz hinweg die Ermittlung zumindest einiger dieser Fehlerzustände.

Besteht beispielsweise ein zumindest teilweiser Verschluss der Eingangsleitung, kann kein Fluid in die Vor- und die Förderkammer einströmen, so dass beim Bewegen des dritten Abschnitts in die Endstellung kein Fluid verdrängt wird und entsprechend kein Druckaufbau in der Förderkammer erfolgt. Dieses Fehlen des Druckaufbaus in der Förderkammer kann über die beschriebene Druckmessung erkannt werden.

Beim Eindringen von Luft in die Pumpe wird aufgrund der Kompressibilität von Luft ebenfalls kein Druckaufbau oder nur ein geringer Druckaufbau in der Förderkammer erzeugt, wenn der dritte Abschnitt in Endstellung gebracht wird. Bei einer verschlossenen oder zumindest teilweise verschlossenen Ausgangsleitung wird hingegen der Ausgangsdruck bei Öffnen des ersten Abschnittes (d. h. beim Bewegen des ersten Abschnitts in seine Ausgangsstellung) ansteigen.

Die Druckmessung in der Förderkammer erfolgt insbesondere nicht-invasiv, wozu z. B. an einer Außenseite des Hohlkörpers ein bewegbares Übertragungselement (z. B. in Form einer Platte) anliegt. Eine Bewegung der Hohlkörperaußenwand, die bei einem Druckanstieg bzw. Druckabfall auftritt, wird von dem Übertragungselement aufgenommen, so dass z.B. Druckbestimmungsmittel der Pumpe anhand der Position des Übertragungselementes der Innendruck in der Förderkammer ermitteln können.

Des Weiteren können Überwachungsmittel vorgesehen sein, die anhand einer Information der Druckbestimmungsmittel und einer Information bezüglich der zum Bewegen des ersten, zweiten, dritten und/oder vierten Abschnitts der flexiblen Wandung erforderlichen Leistung mindestens einen Betriebszustand der Pumpe bestimmen. Beispielsweise lässt sich erkennen, ob die flexible Wandung ein Leck (einen Riss oder einen sonstigen Defekt) aufweist.

Die Erfindung betrifft eine Pumpenanordnung mit einer wie oben beschriebenen Pumpe sowie einem an oder in der Pumpe angeordneten Hohlkörper in Form eines Schlauches, durch den hindurch Fluid förderbar ist, wobei ein Abschnitt der Schlauchwandung eine flexible Wandung des Hohlkörpers bildet, die mit der Aktuatorvorrichtung der Pumpe zusammenwirkt, wie oben erläutert. Mit dieser Pumpenanordnung wird somit eine Schlauchpumpe realisiert, die beispielsweise im medizinischen Bereich oder Analysebereich einsetzbar ist, wo Verunreinigungen des zu fördernden Fluids vermieden werden müssen.

Beispielsweise ist die Schlauchpumpe in Form einer Infusionspumpe ausgebildet, wobei der Schlauch insbesondere als Wegwerfelement (Einmalelement) gestaltet ist, das nach

Gebrauch ausgetauscht werden kann. Die Pumpe weist insbesondere eine Vorrichtung zur Aufnahme des Schlauches auf, so dass er über die Aktuatorvorrichtung gepumpt werden kann, d. h. die Aktuatorvorrichtung vier (insbesondere voneinander beabstandete) Abschnitte der Schlauchwandung bewegen kann, wie oben beschrieben.

Die Erfindung betrifft auch eine Pumpenanordnung mit einer wie oben beschriebenen Pumpe sowie einem an oder in der Pumpe angeordneten Hohlkörper, durch den hindurch Fluid förderbar ist, wobei das innere Volumen des Hohlkörpers zumindest abschnittsweise durch eine flexible Wandung in Form einer Membran begrenzt ist, die mit der Aktuatorvorrichtung der Pumpe zusammenwirkt. Insbesondere ist die Membran ein Bestandteil des Hohlkörpers.

Die Aktuatorvorrichtung der Pumpe wirkt also so mit der Membran des Hohlkörpers zusammen, dass durch das Bewegen von vier Abschnitten der Membran gemäß der Pumpsequenz das in dem Hohlkörper befindliche Fluid gefördert und eine Förderkammer der Pumpe mit einem Vordruck beaufschlagt wird. Insbesondere ist der mit der Membran versehene Hohlkörper in Form einer medizinischen "Kassette" ausgebildet, d. h. einem Einmalartikel, der nach Benutzung entsorgt werden kann. Die Kassette weist bereits die mit der Aktuatorvorrichtung der Pumpe zusammenwirkende flexible Membran auf und wird insbesondere mit einer Zu- und einer Ausgangsleitung (jeweils insbesondere in Form eines Schlauches) mit einem Flüssigkeitsreservoir verbunden. Entsprechend weist die Pumpe Mittel zur Aufnahme der Kassette auf.

Die Kombination aus Kassette und Zu- und Ausgangsleitung wird auch als "Set" bezeichnet, wobei die Membranpumpenanordnung auch Mittel zur Erkennung des Settyps aufweisen kann, um zu erkennen, ob das korrekte Set in die Pumpe eingelegt wurde.

Es wird darauf hingewiesen, dass die Membran auch aus mehreren separaten, insbesondere voneinander beabstandeten Membranelementen bestehen kann. Beispielsweise umfasst die Aktuatorvorrichtung, wie oben beschrieben, eine Mehrzahl von Andrückelementen, wobei jedem der Andrückelemente ein eigenes Membranelement zugeordnet sein kann.

Darüber hinaus bildet der Hohlkörper der Pumpenanordnung zwischen seinem vierten Abschnitt und der Förderkammer - wie oben bereits erwähnt - eine Vorkammer aus und weist Mittel zum Erzeugen einer Kraft auf das Fluid in der Vorkammer auf, wobei die Mittel zum Erzeugen der Kraft einen elastisch ausgebildeten Teilbereich der flexiblen Wandung des Hohlkörpers umfassen. Hierbei ist denkbar, dass die Pumpe wie oben beschrieben zusätzlich oder alternativ ein elastisch gelagertes erstes Andrückelement aufweist. Es ist jedoch insbesondere möglich, dass die Kraft ausschließlich über die Elastizität der flexiblen Wandung erzeugt wird.

Beispielsweise weist die flexible Wandung im Bereich des elastischen Teilbereichs eine geringere Wanddicke und/oder ein Material mit einem geringeren Elastizitätsmodul als im Bereich der Förderkammer auf. Die flexible Wandung ist somit im Bereich der Vorkammer flexibler als im Bereich der Förderkammer, wobei die Rückstellkraft der Wandung im Bereich der Vorkammer für den notwendigen Innendruck zur vollständigen Befüllung der Förderkammer sorgt. Zusätzlich kann auch vorgesehen sein, dass der Abschnitt der flexiblen Wandung im Bereich der Förderkammer über die Aktuatorvorrichtung während des Bewegens des ersten Abschnittes so in Position gehalten wird, dass sich das Volumen der Förderkammer unter dem Einströmen von Fluid zumindest im Wesentlichen nicht vergrößern kann, so dass beim Bewegen des dritten Abschnittes nur eine vordefinierte Menge des Fluids in die Förderkammer einströmen kann, während das Volumen der Vorkammer veränderbar ist.

Die Erfindung betrifft auch einen Hohlkörper, insbesondere in Form eines Schlauches, für eine wie oben beschriebene Pumpe, mit einer flexiblen Wandung mit einem ersten und einem zweiten elastischen Teilbereich, wobei die flexible Wandung im Bereich des ersten elastischen Teilbereichs eine geringere Wanddicke und/oder ein Material mit einem geringeren Elastizitätsmodul als im Bereich des zweiten elastischen Teilbereichs aufweist. Es ist auch denkbar, dass es sich bei dem Hohlkörper um eine Aufnahme handelt, die durch eine Membran begrenzt ist, wobei die Membran die flexible Wandung ausformt und die beschriebenen unterschiedlichen Teilbereiche aufweist. Der erste und der zweiten Teilbereich können einstückig miteinander ausgeformt sein; es ist jedoch auch möglich, dass sie zunächst als separate Teile erzeugt werden, die z.B. stoffschlüssig miteinander verbunden werden.

Des Weiteren betrifft die Erfindung ein Verfahren zum Fördern eines Fluids, mit den Schritten:
- Bereitstellen eines Hohlkörpers, in dem das Fluid aufgenommen wird,
- Bewegen eines ersten, zweiten und dritten Abschnitt einer flexiblen Wandung des Hohlkörpers gemäß einer vorgegebenen und periodisch wiederholten Fördersequenz aus einer Ausgangsstellung in eine Endstellung, wobei
   sich der Strömungsquerschnitt des Hohlkörpers im Bereich des ersten, zweiten oder dritten Abschnitts verringert, wenn der entsprechende Abschnitt in seine Endstellung bewegt wird, so dass der Strömungsquerschnitt des Hohlkörpers im Bereich dieser Abschnitte gemäß der Fördersequenz periodisch verringert und auf diese Weise in dem Hohlkörper Fluid entlang einer Förderrichtung gefördert wird,
- ein Bereich des Hohlkörpers zwischen dem ersten und dem dritten Abschnitt eine Förderkammer ausgebildet, und
- Bewegen eines vierten Abschnitts, der sich entlang der Förderrichtung vor dem dritten Abschnitt befindet, sowie des ersten Abschnitts in die Endstellung, und
- zum Erzeugen eines möglichst reproduzierbaren Druckes in der Förderkammer Bewegen des dritten Abschnitts in seine Endstellung, während sich sowohl der vierte als auch der erste Abschnitt in Endstellung befinden, so dass durch das Bewegen des dritten Abschnitts in die Endstellung Fluid in die Förderkammer gedrückt wird.

Insbesondere wird innerhalb einer Fördersequenz nach dem Bewegen des dritten Abschnitts der flexiblen Wandung in seine Endstellung der erste Abschnitt aus seiner Endstellung in die Ausgangsstellung und gleichzeitig oder anschließend der zweite Abschnitt in Endstellung gebracht, um Fluid aus der Förderkammer herauszudrücken. Natürlich muss die Bewegung des ersten Abschnitts nicht vollständig vor dem Bewegen des zweiten Abschnitts in die Ausgangsstellung abgeschlossen sein. Des Weiteren ist nicht unbedingt notwendig, dass das Bewegen des ersten und des zweiten Abschnittes vollkommen synchron erfolgt, d. h. zum gleichen Zeitpunkt beginnt. Beispielsweise ist denkbar, dass erst mit einer gewissen Zeitverzögerung nach dem Beginn des Bewegens des ersten Abschnitts das Bewegen des zweiten Abschnittes in Endstellung einsetzt.

Die Erfindung wird im Folgenden anhand von Ausführungsbeispielen unter Bezugnahme auf die Figuren näher erläutert. Es zeigen:
- Fig. 1 A-1 F: eine Pumpe gemäß einem ersten Ausführungsbeispiel der Erfindung zu verschiedenen Zeitpunkten während einer Fördersequenz;
- Fig. 2: eine Pumpe gemäß einem zweiten Ausführungsbeispiel der Erfindung;
- Fig. 3: eine Pumpe gemäß einem dritten Ausführungsbeispiel der Erfindung;
- Fig. 4: eine Pumpe gemäß einem vierten Ausführungsbeispiel der Erfindung;
- Fig. 5A, 5B: unterschiedliche Ansichten einer Pumpe gemäß einem fünften Ausführungsbeispiel der Erfindung; und
- Fig. 6A-6E: eine Pumpe gemäß einem sechsten Ausführungsbeispiel der Erfindung zu unterschiedlichen Zeitpunkten während einer Fördersequenz.

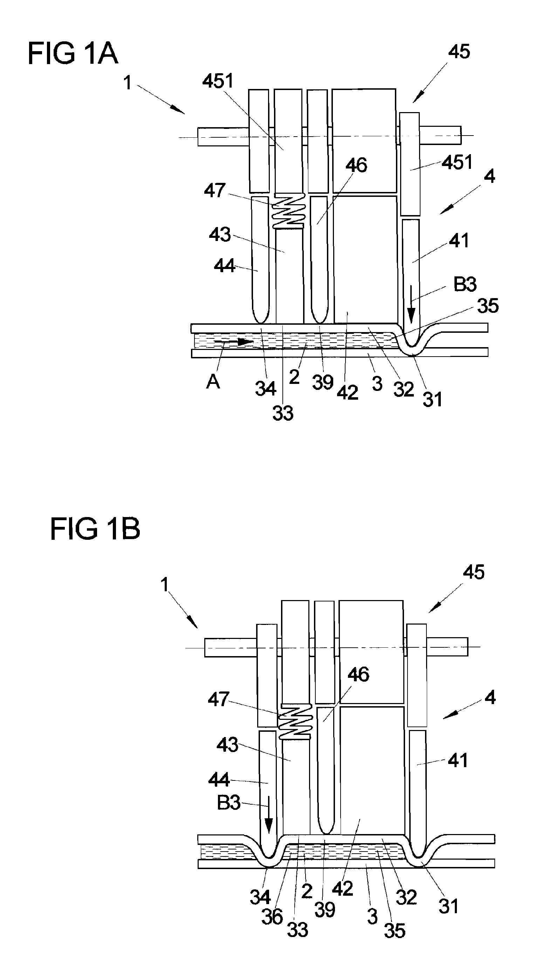

Die in den Figuren 1A bis 1F dargestellte Pumpe 1 dient zum Fördern eines Fluids in Form einer Flüssigkeit 2 durch einen Hohlkörper in Form eines Schlauches 3, d.h. die Pumpe ist als Schlauchpumpe realisiert. Die Pumpe 1 umfasst eine Aktuatorvorrichtung 4 mit vier im Wesentlichen quer zur Längsrichtung des Schlauches 3 im Bereich der Pumpe bewegbaren Andrückelementen in Form von eines ersten, zweiten, dritten und vierten Stößels 41- 44.

Die Stößel 41- 44 sind jeweils einem ersten, zweiten, dritten und vierten Abschnitt 31 - 34 der Schlauchwandung ("flexible Wandung") des Schlauches 3 zugeordnet und über Antriebsmittel in Form einer Nockenwelle 45 bewegbar, wobei sie jeweils in einer im Wesentlichen linearen Bewegung senkrecht zum Schlauch zwischen einer Ausgangsposition und einer Endposition hin und her bewegt werden können. Durch die Bewegung in die Endposition bewegen (drücken) die Stößel 41 - 44 auch den ihnen jeweils zugeordneten Abschnitt 31 bis 34 des Schlauches 3 aus seiner Ausgangsstellung (die einer Stellung des Schlauches ohne Druck des jeweiligen Stößels entspricht) in eine Endstellung, wobei sich der Strömungsquerschnitt des Schlauches 3 im Bereich desjenigen Abschnitts, der in Endstellung bewegt wird, verringert bzw. der Schlauch dort ganz verschlossen wird. Die Reihenfolge, in der die Stößel 41 - 44 in die Endposition bewegt werden, ist insbesondere durch die Ausgestaltung und Anordnung von Nocken 451, die den Stößeln 41 - 44 jeweils zugeordnet sind, der Nockenwelle 45 festgelegt.

Der erste und der vierte Stößel 41, 44 sind (in Förderrichtung A betrachtet) schmaler ausgebildet als der zweite Stößel 42 und dienen in erster Linie zum Sperren bzw. Freigeben des Schlauches 3 (d.h. als Ventilelemente), während der zweite Stößel 42 als Förderstößel arbeitet und zum Ausstoßen von Flüssigkeit aus einer in seinem Bereich (d.h. zwischen dem ersten und dem dritten Stößel 41, 43) gebildeten Förderkammer 35 des Schlauches 3 dient. Genauer wird die Förderkammer 35 durch den ersten Stößel 41 (Auslassventilstößel) und einen zwischen dem zweiten und dem dritten Stößel 42, 43 angeordneten weiteren Stößel 46 begrenzt.

Der weitere Stößel 46 begrenzt darüber hinaus eine sich auf seiner der Förderkammer abgewandten Seite bis zum vierten Stößel 44 (Einlassventilstößel) erstreckende Vorkammer 36. Der Vorkammer 36 ist der erste Stößel 43 ("Vorkammerstößel") zugeordnet, der über ein elastisches Element in Form einer Feder 47 mit einem Nocken 451 der Nockenwelle 45 verbunden ist. Über die Feder 47 wird der Vorkammerstößel 43 in Abhängigkeit von der Position der Nockenwelle 45 gegen den ersten Abschnitt 33 der Schlauchwandung des Schlauches 3 gedrückt und z.B. in seiner Ausgangsposition in Anlage mit der Schlauchwandung gehalten. Es wird darauf hingewiesen, dass sich zwischen der Außenseite der Schlauchwandung und zumindest einigen der Stößel 41-44, 46 eine (z.B. membranartige) Schutzlage (nicht dargestellt) befinden kann, so dass Anlageabschnitte der Stößel über die Schutzlage (d.h. mittelbar) gegen die Schlauchwandung drücken.

Zu Beginn eines Pumpzyklus (d.h. zu Beginn einer Fördersequenz) wird der Auslassventilstößel 41 aus einer Ausgangsposition in eine Sperrposition bewegt (entlang des Pfeils B3), so dass der dem Auslassventilstößel 41 zugeordnete Abschnitt 31 des Schlauches 3 ebenfalls in eine Sperrstellung gebracht und der Schlauch 3 somit in diesem Bereich verschlossen wird. Anschließend wird auch der Einlassventilstößel 44 in die Sperrstellung gebracht, so dass der vierte Schlauchabschnitt 34 ebenfalls in Sperrstellung bewegt und der Schlauch auch in diesem Bereich verschlossen wird (Fig. 1 B).

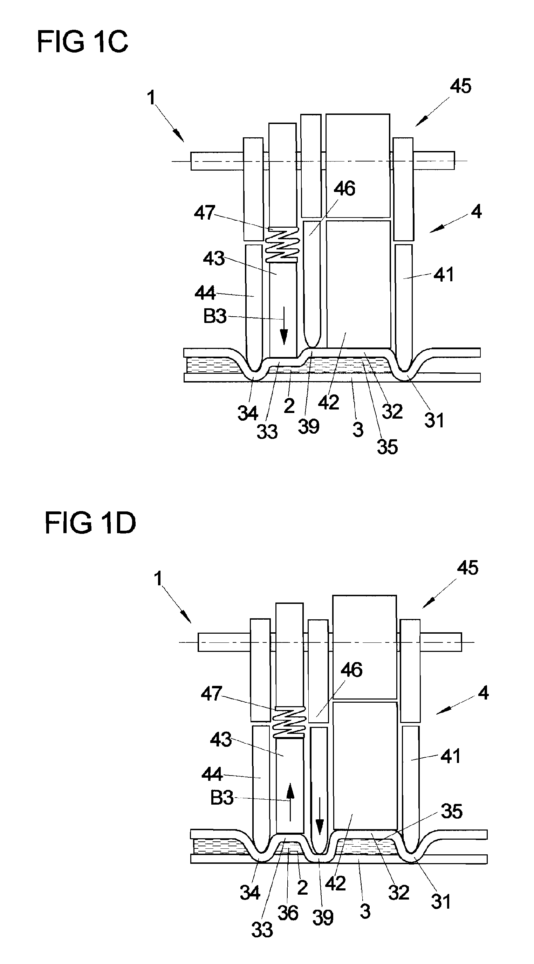

Nach dem Schließen des Auslassventilstößels 41 und des Einlassventilstößels 44 wird auch der Vorkammerstößel 43 in Richtung auf seine Endposition bewegt (Fig. 1 C), so dass er den Schlauch 3 um ein gewisses Maß (jedoch nicht unbedingt vollständig) zusammendrückt, d.h. den Querschnitt im Bereich der Vorkammer verringert, so dass in der Vorkammer 36 befindliche Flüssigkeit in die Förderkammer 35 gedrückt wird. Insbesondere wird der Vorkammerstößel 43 soweit bewegt, bis das verdrängte Fluidvolumen die Förderkammer im Wesentlichen vollständig füllt. Infolgedessen wird in der Förderkammer ein über eine Mehrzahl von Pumpzyklen (Fördersequenzen) reproduzierbarer Vordruck erzeugt, so dass die Förderrate der Pumpe möglichst unabhängig von einem am Pumpeneingang (d.h. vor dem Einlassventilstößel 44) vorhandenen Eingangsdruck ist.

Nach Bewegen des Vorkammerstößels 43 wird der weitere Stößel 46 ebenfalls in seine Endstellung bewegt (Fig. 1D). Hierdurch wird Flüssigkeit im Bereich des Schlauches 3 unterhalb eines Schlauchabschnitts 39, der dem weiteren Stößel 46 zugeordnet ist, verdrängt und insbesondere in die Vorkammer gedrückt. Ein Teil der verdrängten Flüssigkeit wird auch in die Förderkammer einströmen, falls diese durch das Bewegen des Vorkammerstößels 43 noch nicht vollständig gefüllt ist. Es wird darauf hingewiesen, dass auf den weiteren Stößel 46 auch verzichtet werden kann, so dass das Befüllen der Förderkammer nur mittels des Vorkammerstößels 43 erfolgt. Ein Herauslaufen von Flüssigkeit aus der Förderkammer wird in diesem Fall mittels des Vorkammerstößels 43 und des geschlossenen Einlassventilstößels 44 verhindert., Das Volumen der Vorkammer ist aufgrund der elastischen Schlauchwandung und der elastischen Lagerung des Vorkammerstößels 43 veränderbar, so dass in der Vorkammer von dem weiteren Stößel 46 verdrängtes Fluid aufgenommen werden kann und auf diese Weise das Auftreten eines möglicherweise zu einer Beschädigung des Schlauches führenden hohen Druckes beim Schließen des weiteren Stößels 46 möglichst vermieden wird. Mittels der elastischen Lagerung des Vorkammerstößels 43 kann insbesondere auch eine über die Betriebsdauer der Pumpe abnehmende Elastizität des Schlauches im Bereich der Vorkammer kompensiert werden.

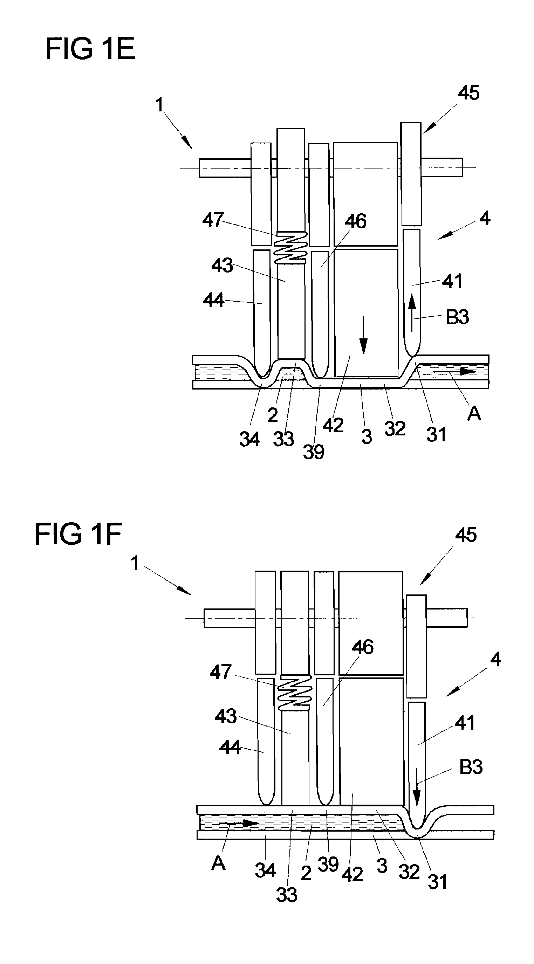

Nachdem der weitere Stößel 46 in die Endposition gebracht wurde, d.h. sich der diesem Stößel zugeordnete Abschnitt 39 des Schlauches 3 ebenfalls in seiner Endstellung befindet und den Schlauch in diesem Bereich sperrt, wird der Auslassventilstößel 41 aus seiner Sperrposition in Richtung Ausgangsstellung bewegt, d.h. der Pumpenausgang wird geöffnet. Gleichzeitig oder anschließend wird der Förderstößel 42 in Endstellung bewegt, so dass die in der Förderkammer 35 befindliche Flüssigkeit entlang der Förderichtung A aus der Kammer und aus der Pumpe hinaus gedrückt wird (Fig. 1 E).

Der Förderzyklus wird dadurch beendet, dass die Pumpe wieder mit Flüssigkeit gefüllt wird, d.h. Flüssigkeit angesaugt wird, wozu bei geschlossenem Auslassventilstößel 41 sämtliche anderen Stößel in ihre Ausgangsposition bewegt werden, so dass sich die bisher in Endstellung befindlichen Abschnitte 32, 33, 34 des Schlauches 3 aufgrund der Schlauchelastizität ebenfalls zurück in ihre Ausgangsstellung bewegen, so dass in diesen Schlauchbereichen ein Unterdruck entsteht und Flüssigkeit angesaugt wird (Fig. 1 F). Anschließend beginnt der Förderzyklus von vorn.

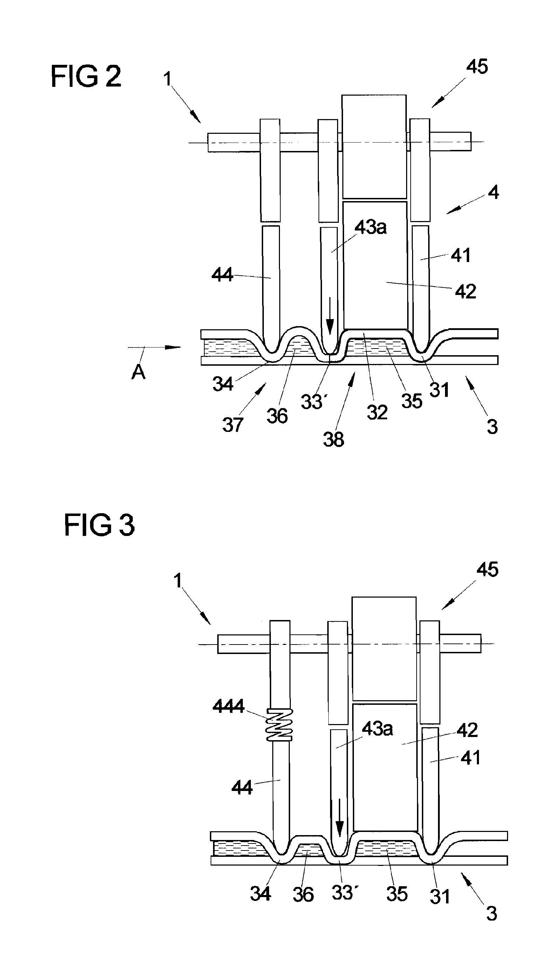

Die Figur 2 betrifft eine zweite Ausführungsform der erfindungsgemäßen Pumpe. Danach übernimmt ein ähnlich dem weiteren Stößel 46 der Figuren 1A bis 1F ausgebildeter dritter Stößel (Ventilstößel 43a) die Vorbefüllung der Förderkammer, d.h. hier bildet der Ventilstößel 43a das "dritte Andrückelement" aus, über das ein zugeordneter dritter Abschnitt 33' des Schlauches bewegt werden kann. Es ist analog zu den Fig. 1A bis 1F zwar ebenfalls ein (entlang der Förderrichtung A betrachtet) vor dem ersten Ventilstößel 43a angeordneter Einlassventilstößel 44 vorhanden, so dass zwischen dem Einlassventilstößel 44 und dem Ventilstößel 43a eine Vorkammer 36 und zwischen dem Ventilstößel 43a und dem Auslassventilstößel 41 eine Förderkammer 35 ausgebildet wird. Allerdings wird auf den elastisch gelagerten Vorkammerstößel 43 (der in dem Ausführungsbeispiel der Figuren 1A bis 1F das "dritte Andrückelement" ausbildet) verzichtet.

Es sei angemerkt, dass der Ventilstößel 43a natürlich nicht zwingend identisch zu dem weiteren Stößel 46 der Figuren 1A bis 1F ausgebildet und angeordnet sein muss. Beispielsweise kann der Ventilstößel 43a eine andere Breite (entlang der Förderrichtung betrachtet) und/oder mit einem anderen Abstand zu dem Einlassventilstößel 44 und/oder dem Verdrängerstößel 42 angeordnet sein.

Der Schlauch 3 ist so ausgebildet, dass er im Bereich der Vorkammer 36 einen ersten Bereich 37 besitzt, der eine höhere Elastizität aufweist als ein zweiter Bereich 38 im Bereich der Förderkammer 35. Beispielsweise weist der erste Bereich 37 des Schlauches 3 eine geringere Wanddicke und/oder ein geringeres Elastizitätsmodul auf als der zweite Bereich 38. Somit kann Flüssigkeit, die beim Bewegen des Ventilstößel 43a in die Endposition verdrängt wird, durch den sich ausdehnenden elastischeren ersten Bereich 37 des Schlauches 3, der die Vorkammer 36 ausbildet, aufgenommen werden, wodurch einem Bersten des Schlauches entgegengewirkt wird.

Des Weiteren ist möglich, dass ein elastisches Ausdehnen auch der Förderkammer dadurch verhindert oder einem solchen zumindest entgegengewirkt wird, dass, bevor der Ventilstößel 43a in Sperrposition bewegt wird, der Förderstößel 42 in eine Position gebracht wird, in der er an der Schlauchwandung anliegt, wie bereits weiter oben erläutert.

Des Weiteren können zusätzliche Mittel vorgesehen sein, die den Schlauch im Bereich der Förderkammer 35 umgreifen und somit das Ausdehnen der Förderkammer aufgrund der Schlauchelastizität auch in diesem Bereich entgegenwirken. Beispielsweise sind diese Mittel an dem Förderstößel 42 angeordnet bzw. der Förderstößel 42 ist entsprechend so ausgeformt, dass er in seiner Endposition zumindest einen Teil des zweiten Schlauchabschnitts 32 umgreift.

Beispielsweise kann die Fördergenauigkeit der Pumpe, d. h. die Genauigkeit, mit der ein bestimmtes Fluidvolumen pro Zeiteinheit gefördert wird, um einen Faktor, der sich aus dem Verhältnis des Elastizitätsmoduls des ersten Abschnitts des Schlauches und des Elastizitätsmoduls des zweiten Abschnitt des Schlauches und/oder dem Verhältnis der jeweiligen Schlauchdicken ergibt, erhöht sein.

Figur 3 betrifft eine Abwandlung der Figur 2, wonach die Pumpe 1 wiederum einen stromabwärts vor dem dritten Stößel 43a angeordneten Einlassventilstößel 44 aufweist. In diesem Ausführungsbeispiel ist der Einlassventilstößel 44 über ein elastisches Element in Form einer Feder 444 an der Nockenwelle 45 gelagert, so dass er in Sperrposition (oder auch in einer sonstigen Stellung zwischen der Ausgangsposition und der Endposition) bei Überschreiten eines vorgebbaren (insbesondere durch die Federkonstante der Feder 444 bestimmten) Innendruckes in dem Schlauch 3 aus der Sperrstellung weg (in Richtung Ausgangsstellung) bewegt wird, so dass sich der Strömungsquerschnitt des Schlauches im Bereich des Einlassventilstößels 44 vergrößert und Fluid entgegen der Förderrichtung abfließen kann.

Somit ist es möglich, dass Flüssigkeitsvolumen, das weder von der Förderkammer 35 noch von der Vorkammer 36 aufgenommen werden kann, aus dem Pumpbereich (d. h. den Bereich zwischen dem Einlassventilstößel 44 und dem Auslassventilstößel 41) abfließen kann und eine Beschädigung des Schlauches vermieden wird. Die Federkonstante der Feder 444 ist insbesondere auf den jeweils benutzten Schlauch (d. h. insbesondere auf dessen Elastizität und Wandstärke) abgestimmt. Obwohl in Figur 3 im Bereich der Vorkammer kein Vorkammerstößel angeordnet ist, ist es natürlich auch denkbar, dass ein solcher verwendet wird.

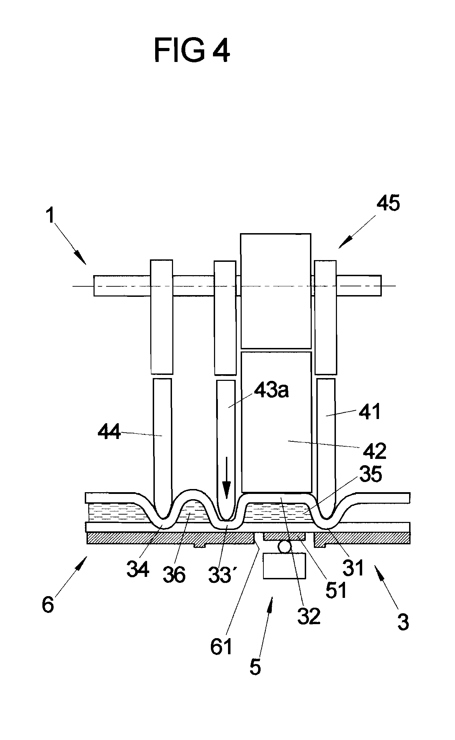

Figur 4 zeigt eine weitere Ausgestaltung der erfindungsgemäßen Pumpe 1, wonach im Bereich der Förderkammer 35 Druckbestimmungsmittel 5 zum Bestimmen eines Innendrucks in der Förderkammer angeordnet sind. Die Druckbestimmungsmittel 5 weisen ein bewegbares Element in Form einer bewegbaren Platte 51 auf, die an einer Außenwand des Schlauches im Bereich der Förderkammer 35, d. h., im Bereich des zweiten Abschnitts 32 des Schlauches, anliegt.

Der Schlauch 3 ist hierbei an einer den Stößeln 41 bis 44 abgewandten Seite an einem Gegenlager 6 gelagert, wobei das Gegenlager 6 im Bereich der Förderkammer 35 eine Aussparung 61 aufweist, in der die bewegbare Platte 51 angeordnet ist. In Abhängigkeit vom Innendruck in der Förderkammer 35 wird sich die Schlauchwandung im Bereich der Förderkammer bewegen, wobei diese Bewegung der Schlauchwandung durch die an der Schlauchwandung anliegende bewegbare Platte 51 aufgenommen und anhand der Position der bewegbaren Platte der Innendruck in der Förderkammer ermittelt wird. Die bewegbare Platte kann z. B. in an sich bekannter Art mit einer Feder (nicht dargestellt) verbunden sein. Selbstverständlich sind zusätzlich oder alternativ auch andere Möglichkeiten zur Druckmessung in der Förderkammer möglich, z. B. über an der Schlauchwandung angebrachte Dehnmessstreifen oder über kapazitive und/oder induktive Messungen.

Die Druckmessung kann insbesondere dazu dienen, Fehlerzustände der Pumpe im Betrieb zu erkennen, z. B. ein Verschluss der Eingangs- und/oder der Ausgangsleitung oder das Vorhandensein von Luft im Pumpenbereich, wie weiter oben bereits erläutert. Des Weiteren ist denkbar, dass die Druckmessung herangezogen wird, um eine aktuelle Förderrate der Pumpe zu ermitteln, wobei auf Basis der ermittelten Förderrate auch eine Regelung der Förderrate erfolgen kann.

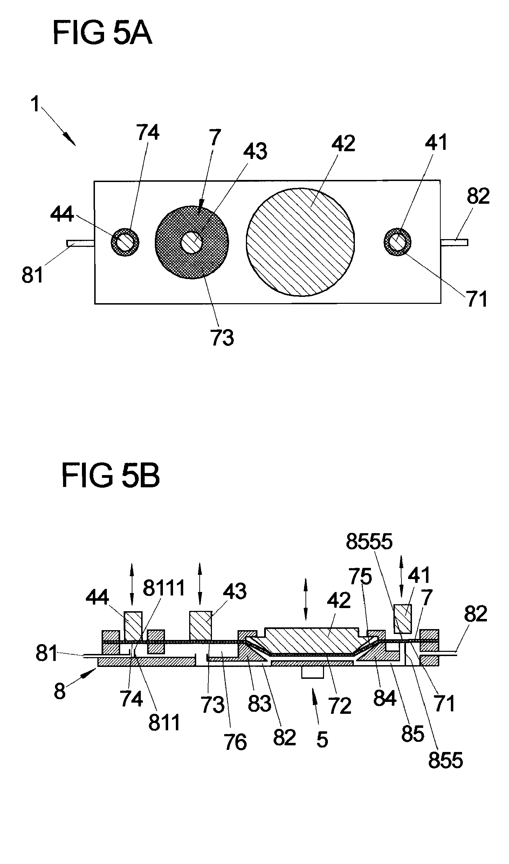

Die Figuren 5A (Draufsicht) und 5B (Schnitt) beziehen sich auf eine weitere Ausführungsform der erfindungsgemäßen Pumpe, wonach die Pumpe in Form einer Membranpumpe ausgebildet ist. Bei dieser Pumpenform ist der Hohlkörper, durch den hindurch Fluid gefördert wird, kein Schlauch, sondern eine Aufnahme 8, die eine als Membran 7 ausgebildete flexible Wandung aufweist.

Die Pumpe weist analog zu den vorhergehenden Beispielen Andrückelemente in Form von Stößeln 41 bis 44 auf, die z. B. ebenfalls analog zu den vorhergehenden Beispielen über eine Nockenwelle (nicht dargestellt) angetrieben werden können, um eine Hin- und Herbewegung der Stößel senkrecht zu der Membranfläche zu erzeugen. Entsprechend werden durch die Bewegung der Stößel 41 bis 44 jeweils den Stößeln zugeordnete Membranabschnitte 71 bis 74 aus einer Ausgangsstellung in eine Endstellung bewegt.

Die Aufnahme 8 ist insbesondere in Form einer so genannten "Kassette" für medizinische Zwecke ausgebildet, die in eine an die Kassette angepasste Aufnahmevorrichtung (nicht dargestellt) der Pumpe eingelegt wird.

Die Aufnahme 8 weist einen Einlasskanal 81 auf, durch den die zu fördernde Flüssigkeit in den Pumpenbereich angesaugt werden kann sowie einen Auslasskanal 82, aus dem die Flüssigkeit aus der Aufnahme ausgestoßen wird. Der Einlasskanal weist einen näherungsweise senkrecht zu dem (vierten) Membranabschnitt 74 orientierten Endbereich 811 auf, in dem eine Ausströmöffnung 8111 vorgesehen ist. Unterhalb des Förderstößels 42 ist analog zu der oben beschrieben Schlauchpumpe eine Förderkammer 75 ausgebildet, die zumindest zum Teil von Seitenwänden 83, 84 der Aufnahme 8 begrenzt ist. Die Förderkammer 75 erstreckt sich somit in dem Beispiel der Figuren 5A, 5B nur über einen Teil des Abstandes zwischen dem ersten und dem dritten Stößel 41, 43. Von der Förderkammer 75 erstreckt sich ein weiterer Kanal 85 in den Bereich des dritten Membranabschnitts 73 hinein, wobei der Kanal 85 eine senkrecht zu diesem Membranabschnitt orientieren Endabschnitt 855 mit einer Ausströmöffnung 8555 aufweist.

Mittels des Kanals 85 (insbesondere mittels seines senkrecht zur Membran verlaufenden Endes 855) und des ersten Membranabschnitts 71 wird ein "Anti-free-flow-Ventil" realisiert, das ein freies Ausströmen der Flüssigkeit aus der Pumpe heraus verhindert. Erst, wenn der Innendruck in der Pumpe einen vorgebbaren Minimalwert übersteigt, wird der Membranabschnitt 71 von dem Ende 855 weg bewegt und das Ventil geöffnet. Zusätzlich wirkt die Anordnung aus dem Kanal 85 und dem dritten Membranabschnitt 71 als Rückschlagventil, das ein Einströmen von Flüssigkeit in die Pumpe entgegen der Förderrichtung verhindert.

Bei Betrieb der Membranpumpe 1 werden analog zu der den Beispielen der Figuren 1 bis 4 zunächst der Einlassventilstößel 44 und der Auslassventilstößel 41 in ihre jeweilige Sperrstellung bewegt. In Sperrstellung liegen die zugeordneten Membranabschnitte 74, 71 dann jeweils der Ausströmöffnung 8111 des Einlasskanals 81 bzw. der Ausströmöffnung 8555 des Auslasskanals 82 an, so dass der Einlass- und der Auslasskanal verschlossen ist, d.h. der Strömungsquerschnitt der Aufnahme 8 (zwischen dem Membranabschnitt 74 und dem Ende 811 bzw. zwischen dem ersten Membranabschnitt 71 und dem Ende 855 des Kanals 85) im Bereich des vierten und des ersten Membranabschnittes wird auf zumindest annähernd null reduziert.

Abschließend wird der dritte Stößel 43 in die Sperrstellung gebracht, so dass ein Teil der unterhalb des ersten Stößels 43 in einer Vorkammer 76 befindlichen Flüssigkeit in Richtung der unterhalb des Förderstößels 42 ausgebildeten Förderkammer 75 gedrückt und somit analog zu der oben beschriebenen Schlauchpumpe ein Vordruck in der Förderkammer erzeugt wird. Vor- und Förderkammer stehen über einen (z.B. ein in der Aufnahme 8 ausgeformten) Kanal 82 zwischen der Vorkammer 76 und der Förderkammer 75 in Strömungsverbindung.

Es ist möglich, dass über die Kraft, mit der der Förderstößel 42 gehalten oder bewegt wird, der Innendruck in der Förderkammer ermittelt wird. Beispielsweise kann die Kraft, mit der der Stößel 42 bewegt wird, anhand der Motorstrom-Kennlinie, mit der ein Antrieb (d. h. die Nockenwelle) der Stößel angetrieben wird, bestimmt werden. Zusätzlich oder alternativ ist es auch möglich, einen separaten Drucksensor im Bereich der Förderkammer vorzusehen; beispielsweise in Form eines bewegbaren Elementes, das an einer Au-βenwand der Aufnahme 8 anliegt.

Bei Verwendung eines separaten Drucksensors 5 ist es zudem möglich, dass aus der Zusammenschau des Drucksensorsignals und der Motorstrom- und/oder Zeit-Phasen-Kennlinie des Motors zum Antrieb der Nockenwelle Betriebszustände und/oder Fehlerzustände der Pumpe oder der Kassette (d. h. der Kombination aus Aufnahme 8 und Membran 7) bestimmt werden. Insbesondere ist es auch möglich, über einen separaten Drucksensor (oder auch über die Kraft zum Bewegen des Förderstempels 42, wie oben beschrieben) ein Leck (Riss oder ein sonstiger Defekt) der Membran und/oder der Aufnahme 8 zu erkennen. Der Drucksensor kann zudem ausgebildet sein, zu detektieren, ob die Kassette 7, 8 korrekt in die Pumpe eingelegt wurde und/oder ob die korrekte Kassette für die beabsichtigte Anwendung eingesetzt wurde.

Des Weiteren ist denkbar, zusätzliche Sensoren vorzusehen, die ebenfalls die Ermittlung von Betriebszuständen der Pumpe ermöglichen; z. B. Ultraschallsensoren oder optische Sensoren.

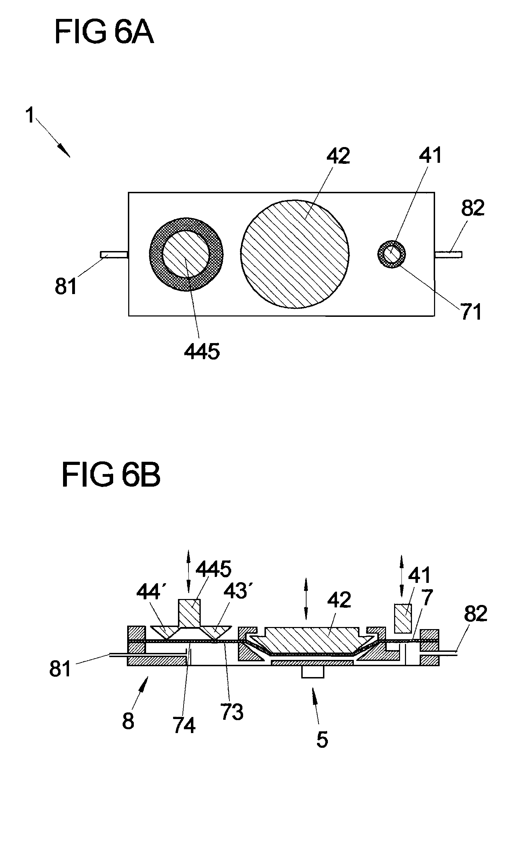

Eine Abwandlung der Membranpumpe aus den Figuren 5A und 5B zeigen die Figuren 6A bis 6E. Hierbei sind der Einlassventilstößel und der dritte Stößel miteinander (z. B. einstückig) verbunden, so dass sie z. B. über einen gemeinsamen Nocken einer Nockenwelle (nicht dargestellt) angetrieben werden können. Genauer sind anstelle separater Stößel in Förderrichtung beabstandete Druckelemente 44', 43' an einem gemeinsamen Aktuatorschaft 445 vorgesehen.

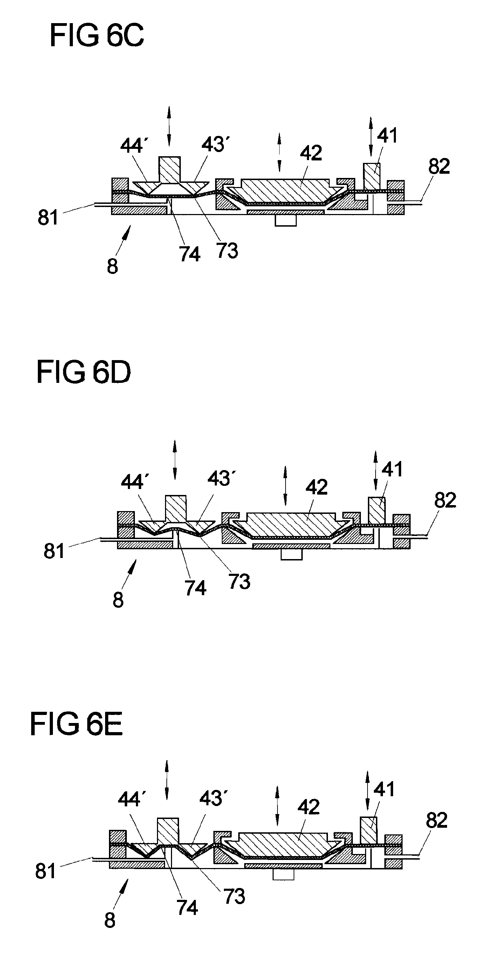

Zu Beginn eines Förderzyklus wird der Auslassventilstößel 41 in seine Sperrstellung gebracht und auch der Schaft 445, d. h. die Druckelemente 43', 44' in ihre Sperrstellung bewegt. Der dem Druckelement 44' zugeordnete (vierte) Membranabschnitt 74 erreicht seine Endstellung, wenn er an einer Ausströmöffnung 8555 des Einlasskanals 81 anliegt und diesen verschließt (Figur 6C). Es wird angemerkt, dass sich der mit dem Druckelement 44' bewegte Membranabschnitt 74 etwas in Richtung des (ersten) Membranabschnitts 71 erstreckt, um die Ausströmöffnung 8555, die sich etwa mittig zwischen dem beiden Druckelementen 44', 43' befindet, abdecken zu können.

Der Aktuatorschaft 445 wird weiter nach unten (d. h. in Richtung auf die Aufnahme 8) bewegt, so dass sich die Membran unterhalb des Druckelementes 43' weiter deformiert (d.h. in die Endstellung gebracht wird), wodurch Flüssigkeit unterhalb des ersten Membranabschnitts 71 in die Förderkammer gedrückt wird (Figur 6D).

Figur 6E zeigt die Endposition des Aktuatorschafts 455 und damit auch des ersten Membranabschnitts 71. Hier liegt der Membranabschnitt 74 fest auf dem Ende des Einlasskanals 81 auf, so dass die Ausströmöffnung 8555 möglichst dicht verschlossen ist, während gleichzeitig Flüssigkeit in die Förderkammer gedrückt wird. Vor dem festen Anliegen des Membranabschnitts 74 (Figur 6D) könnte, falls der Druck in der Förderkammer und unterhalb des Druckelementes 43' zu hoch werden sollte, Flüssigkeit noch entgegen der Förderrichtung über den Einlasskanal 81 aus der Pumpe abströmen.

Es wird darauf hingewiesen, dass Elemente der zuvor anhand der in den Figuren 1 bis 6 beschriebenen Ausführungsbeispiele natürlich auch in Kombination miteinander verwendet werden können. Beispielsweise kann im Ausführungsbeispiel der Figuren 1A bis 1F oder der Figuren 5 und 6 ein Drucksensor 5 gemäß der Figur 4 verwendet werden. Des Weiteren kann z.B. der Antrieb der Stößel 41 bis 44 der Figuren 5 und 6 analog zum Antrieb 45 der Figuren 1 bis 4 gestaltet sein. Auch kann die Membran 7 der Figuren 5 und 6 im Bereich der Vorkammer elastischer als im Bereich der Förderkammer ausgebildet sein. Darüber hinaus kann in allen Ausführungsbeispielen eine Schutzlage zwischen dem Hohlkörper (Schlauch bzw. Membran) und den Andrückelementen vorgesehen sein.

### Bezugszeichenliste

- 1: Pumpe
- 2: Flüssigkeit
- 3: Schlauch
- 4: Aktuatorvorrichtung
- 5: Drucksensor
- 6: Gegenlager
- 7: Membran
- 8: Aufnahme
- 31: erster Abschnitt
- 32: zweiter Abschnitt
- 33, 33': dritter Abschnitt
- 34: vierter Abschnitt
- 35: Förderkammer
- 36: Vorkammer
- 37: erster Schlauchabschnitt
- 38: zweiter Schlauchabschnitt
- 39: Schlauchabschnitt
- 41: erster Stößel
- 42: Förderstößel
- 43, 43a: Auslassventilstößel
- 44: Einlassventilstößel
- 43', 44': Druckelement
- 45: Nockenwelle
- 46: Druckelement
- 47: Feder
- 51: bewegbare Platte
- 61: Aussparung
- 71: erster Membranabschnitt
- 72: zweiter Membranabschnitt
- 73: dritter Membranabschnitt
- 74: vierter Membranabschnitt
- 75: Förderkammer
- 76: Vorkammer
- 81: Einlasskanal
- 82: Auslasskanal
- 85: Kanal
- 444: Feder
- 445: Aktuatorschaft
- 451: Nocken
- 811: Ende Einlasskanal
- 855: Ende Kanal
- 8111, 8555: Ausströmöffnung

## Patentansprüche

1. Pumpe zum Fördern eines Fluids in einem Hohlkörper, mit
- einer Aktuatorvorrichtung (4), die bei Betrieb der Pumpe (1) einen ersten, zweiten und dritten Abschnitt (31-33, 33', 71-73) einer flexiblen Wandung des Hohlkörpers (3, 7) gemäß einer vorgegebenen und periodisch wiederholten Fördersequenz aus einer Ausgangsstellung in eine Endstellung bewegt, wobei
- sich der Strömungsquerschnitt des Hohlkörpers (3, 7) im Bereich des ersten, zweiten oder dritten Abschnitts (31-33, 33', 71-73) verringert, wenn der entsprechende Abschnitt in seine Endstellung bewegt wird, so dass der Strömungsquerschnitt des Hohlkörpers (3, 7) im Bereich dieser Abschnitte gemäß der Fördersequenz periodisch verringert und auf diese Weise in dem Hohlkörper Fluid (2) entlang einer Förderrichtung (A) gefördert wird, und wobei
- ein Bereich des Hohlkörpers (3, 7) zwischen dem ersten und dem dritten Abschnitt (31, 71, 33, 33', 73) der flexiblen Wandung eine Förderkammer (35, 75) darstellt, aus der durch Bewegen des zweiten Abschnitts (32, 72) in die Endstellung Fluid (2) herausgedrückt wird,
**dadurch gekennzeichnet, dass**
die Aktuatorvorrichtung (1) ausgebildet ist,
- einen vierten Abschnitt (34, 74) der flexiblen Wandung, der sich entlang der Förderrichtung (A) vor dem dritten Abschnitt (33, 33',73) befindet, aus einer Ausgangsstellung in eine Endstellung zu bewegen, und
- zum Erzeugen eines möglichst reproduzierbaren Druckes in der Förderkammer (35) den dritten Abschnitt (33, 33', 73) in Endstellung zu bewegen, während sich sowohl der vierte als auch der erste Abschnitt (31, 71, 31, 71) in Endstellung befinden, so dass durch das Bewegen des dritten Abschnitts (33, 33', 73) in die Endstellung Fluid (2) in die Förderkammer (35, 75) gedrückt wird.

2. Pumpe nach einem der vorhergehenden Ansprüche, **dadurch gekennzeichnet, dass** die Aktuatorvorrichtung (4) ein erstes, zweites, drittes und viertes Andrückelement (41, 42, 43, 43a, 43', 44, 44') aufweist, die jeweils dem ersten, zweiten, dritten und vierten Abschnitt zugeordnet sind, sowie Antriebsmittel, die bei Betrieb der Pumpe (1) die Andrückelemente gegen die flexible Wandung drücken, um den ersten, zweiten, dritten und/oder vierten Abschnitt (31-34, 33', 71-74) der flexiblen Wandung zu bewegen.

3. Pumpe nach Anspruch 2, **dadurch gekennzeichnet, dass** zwischen dem zweiten und dem dritten Andrückelement (42, 43) ein weiteres Andrückelement (46) angeordnet ist, das nach Bewegen des dritten Andrückelementes (43) in die Endstellung gebracht wird, wobei die Förderkammer durch das weitere Andrückelement (46) und das erste Andrückelement (41) begrenzt ist.

4. Pumpe nach Anspruch 2 oder 3, **dadurch gekennzeichnet, dass** das dem dritten Abschnitt (33) zugeordnete dritte Andrückelement (43) über ein elastisches Element (47) gelagert ist.

5. Pumpe nach Anspruch 4, **dadurch gekennzeichnet, dass** es sich bei dem elastischen Element (47) um eine Feder handelt.

6. Pumpe nach Anspruch 2, **dadurch gekennzeichnet, dass** die Förderkammer durch das erste und das dritte Andrückelement (41, 43a) begrenzt ist.

7. Pumpe nach einem der Ansprüche 3 bis 6, **dadurch gekennzeichnet, dass** das zweite Andrückelement (42) an dem Hohlkörper anliegt, während das dritte Andrückelement (43, 43a) in seine Endstellung bewegt wird, und/oder sich das zweite Andrückelement (42) entlang der Förderrichtung über mindestens 70%, mindestens 85% oder mindestens 95 % des Abstandes zwischen den die Förderkammer begrenzenden Andrückelementen erstreckt.

8. Pumpe nach einem der Ansprüche 2 bis 7, **dadurch gekennzeichnet, dass** das vierte Andrückelement (44, 44') über ein elastisches Element (444) gelagert ist, so dass es in Endstellung bei Überschreiten eines vorgebbaren Innendruckes in dem Hohlkörper (3, 7) aus der Endstellung weg bewegt wird, so dass sich der Querschnitt des Hohlkörpers im Bereich des vierten Andrückelements (44, 44') vergrößert.

9. Pumpe nach einem der vorhergehenden Ansprüche, **gekennzeichnet durch** Mittel (5) zum Bestimmen des Innendrucks in der Förderkammer (35).

10. Pumpenanordnung mit einer Pumpe nach einem der vorhergehenden Ansprüche sowie einem an oder in der Pumpe angeordneten Hohlkörper in Form eines Schlauches, durch den hindurch Fluid förderbar ist, wobei ein Abschnitt der Schlauchwandung eine flexible Wandung des Hohlkörpers bildet, die mit der Aktuatorvorrichtung der Pumpe zusammenwirkt.

11. Pumpenanordnung mit einer Pumpe nach einem der Ansprüche 1 bis 9 sowie einem an oder in der Pumpe angeordneten Hohlkörper, durch den hindurch Fluid förderbar ist, wobei das innere Volumen des Hohlkörpers zumindest abschnittsweise durch eine flexible Wandung in Form einer Membran begrenzt ist, die mit der Aktuatorvorrichtung der Pumpe zusammenwirkt.

12. Pumpenanordnung, insbesondere nach Anspruch 10 oder 11, mit
- einer Pumpe (1) nach einem der Ansprüche 1 bis 9, wobei zwischen dem vierten und dem dritten Abschnitt (34, 74, 33, 33', 73) des Hohlkörpers (3, 7) eine Vorkammer (36, 76) ausgebildet ist; und
- Mitteln zum Erzeugen einer Kraft auf das Fluid (2) in der Vorkammer (36, 76), wobei
- die Mittel zum Erzeugen der Kraft einen elastisch ausgebildeten Teilbereich (37) der flexiblen Wandung des Hohlkörpers (3, 7) umfassen.

13. Pumpenanordnung nach Anspruch 12, **dadurch gekennzeichnet, dass** die flexible Wandung im Bereich des elastischen Teilbereichs eine geringere Wanddicke und/oder ein Material mit einem geringeren Elastizitätsmodul als im Bereich der Förderkammer aufweist.

14. Hohlkörper, insbesondere in Form eines Schlauches, zur Verwendung mit einer Pumpe gemäß einem der Ansprüche 1 bis 9, **gekennzeichnet durch** eine flexible Wandung mit einem ersten und einem zweiten elastischen Teilbereich (37, 38), wobei die flexible Wandung im Bereich des ersten elastischen Teilbereichs (37) eine geringere Wanddicke und/oder ein Material mit einem geringeren Elastizitätsmodul als im Bereich des zweiten elastischen Teilbereichs (38) aufweist.

15. Verfahren zum Fördern eines Fluids, mit den Schritten:
- Bereitstellen eines Hohlkörpers (3, 7), in dem das Fluid (2) aufgenommen wird,
- Bewegen eines ersten, zweiten und dritten Abschnitt (31-33, 33', 71-73) einer flexiblen Wandung des Hohlkörpers (3, 7) gemäß einer vorgegebenen und periodisch wiederholten Fördersequenz aus einer Ausgangsstellung in eine Endstellung, wobei
sich der Strömungsquerschnitt des Hohlkörpers (3, 7) im Bereich des ersten, zweiten oder dritten Abschnitts (31-33, 33', 71-73) verringert, wenn der entsprechende Abschnitt in seine Endstellung bewegt wird, so dass der Strömungsquerschnitt des Hohlkörpers (3, 7) im Bereich dieser Abschnitte gemäß der Fördersequenz periodisch verringert und auf diese Weise in dem Hohlkörper Fluid entlang einer Förderrichtung (A) gefördert wird, und wobei
- ein Bereich des Hohlkörpers (3, 7) zwischen dem ersten und dem dritten Abschnitt (31, 71, 33, 33', 73) eine Förderkammer (35, 75) ausgebildet,
**gekennzeichnet durch**
- Bewegen eines vierten Abschnitts (34, 74), der sich entlang der Förderrichtung (A) vor dem dritten Abschnitt (33, 33', 73) befindet, sowie des ersten Abschnitts (31, 71) in die Endstellung, und
- zum Erzeugen eines möglichst reproduzierbaren Druckes in der Förderkammer (35, 75) Bewegen des dritten Abschnitts (33, 33', 73) in seine Endstellung, während sich sowohl der vierte (34, 74) als auch der erste Abschnitt (31, 71) in Endstellung befinden, so dass **durch** das Bewegen des dritten Abschnitts (33, 33', 73) in die Endstellung Fluid (2) in die Förderkammer (35, 75) gedrückt wird.
